Europäisches Patentamt

European Patent Office

Office européen des brevets

(19)

(11) Veröffentlichungsnummer: **0 151 686**
**B1**

(12) **EUROPÄISCHE PATENTSCHRIFT**

(45) Veröffentlichungstag der Patentschrift: **04.04.90**

(21) Anmeldenummer: **84111884.7**

(22) Anmeldetag: **04.10.84**

(51) Int. Cl.⁵: **A 61 C 13/00,** A 61 K 6/08,
A 61 L 15/07

(54) Verfahren zur Herstellung von kieferorthopädischen Geräten und Apparaturen.

(30) Priorität: **11.02.84 DE 3404904**

(43) Veröffentlichungstag der Anmeldung:
**21.08.85 Patentblatt 85/34**

(45) Bekanntmachung des Hinweises auf die
Patenterteilung:
**04.04.90 Patentblatt 90/14**

(84) Benannte Vertragsstaaten:
**AT CH DE FR GB LI SE**

(56) Entgegenhaltungen:
**EP-A-0 018 672**
**EP-A-0 110 193**
**DE-B-1 516 456**
**FR-A-2 182 091**
**GB-A-2 018 666**

(73) Patentinhaber: **Kulzer GmbH**
**Philipp-Reis-Strasse 8**
**D-6393 Wehrheim (TS.)1 (DE)**

(72) Erfinder: **Angrick, Michael, Dr.**
**Richard-Wagner-Strasse 45**
**D-1000 Berlin 10 (DE)**
Erfinder: **Trautwein, Ullrich**
**Philipp-Reis-Strasse 8**
**D-6393 Wehrheim (DE)**
Erfinder: **Lenz, Kurt**
**Philipp-Reis-Strasse 8**
**D-6393 Wehrheim (DE)**

(74) Vertreter: **Heinen, Gerhard, Dr.**
**Heraeus Holding GmbH Heraeusstrasse 12-14**
**D-6450 Hanau am Main (DE)**

EP 0 151 686 B1

Courier Press, Leamington Spa, England.

# EP 0 151 686 B1

## Beschreibung

Die Erfindung betrifft ein Verfahren zur Herstellung von kieferorthopädischen Geräten und Apparaturen durch Aufbringen eines monomere Acrylsäureester und/oder Methacrylsäureester enthaltenden Materials auf ein Gipsmodell und Polymerisation des Materials.

Kieferorthopädische Geräte und Apparaturen dienen der Gebißregulierung und der Beseitigung von Zahnstellungsanomalien. Sie können, soweit sie aus Kunststoff auf Acrylat- beziehungsweise Methacrylat-Basis bestehen, durch Aufbringen von pulverförmigem Polyacrylat beziehungsweise Polymethacrylat und flüssigem Monomer, meist Methylmethacrylat, auf Gipsmodelle nach dem Tropfverfahren oder unter Anwendung der Sprühtechnik oder der Modelliertechnik und anschließende Polymerisation bei erhöhter Temperatur unter Druck hergestellt werden.

Aus einer Mehrzahl von im Tiefziehverfahren geformten und in mehreren Lagen aufeinanderliegenden sowie durch Klebeschichten miteinander verbundenen Kunststoff-Platten bestehende Zahnprothesen, keiferorthopädische Apparaturen und dergleichen werden in dem deutschen Gebrauchsmuster 6 753 339 beschrieben.

Ein auf dem Gebiet der Zahnprothetik angewandtes Verfahren zur Herstellung von Zahnersatz ist das des schichtweisen Aufbringens von polymerisierbarem Material auf eine Gipsform und der Polymerisation durch Erwärmen, Bestrahlung mit sichtbarem Licht oder UV-Licht oder auf andere Weise (siehe zum Beispiel DE—B—1 516 456 und DE—A—2 910 077).

Bei dem in DE—B—1 516 456 beschriebenen Verfahren zur Herstellung von Zahnersatzteilen durch schichtweises Auftragen von polymerisierbaren Massen und Polymerisation erfolgt die Teilpolymerisation bei einer bestimmten, je nach Art des Monomers festzulegenden Temperatur durch Wärme, Strahlung oder Hochfrequenz, die Fertigpolymerisation meist mit den angeführten einfachen Hilfsmitteln (Flamme, Heißluft, Infrarotlampe und dergleichen).

Die EP—A—0 110 193, welche einen Stand der Technik gemäß Art 54(3)(4) darstellt, betrifft ein Verfahren zur Herstellung individueller Metallgußteile unter Verwendung eines mindestens teilweise durch Photopolymerisation einer photopolymerisierbaren Masse aufgebauten Gußmodells. Das Gußmodell kann durch schrittweisen Aufbau in dünnen Schichten, die unter Lichteinwirkung sofort zu einem harten, formstabilen Kunststoff erhärten, hergestellt werden. Es ist vorteilhaft, sichtbares Licht im Wellenlängenbereich von etwa 400—500 nm für die Photopolymerisation zu verwenden; jedoch sind auch kürzerwellige Strahlungsbereiche möglich.

In EP—A—0 018 672 wird ein durch Bestrahlung mit UV-Licht härtbares Material für Überzüge beschrieben, das das Addukt aus einem Hydroxyester der Acryl- oder Methacrylsäure und einem Di-, Tri- oder Tetraisocyanat enthalten kann.

Es ist die Aufgabe der Erfindung, ein Verfahren zur Herstellung von kieferorthopädischen Geräten und Apparaturen durch Aufbringen eines polymerisierbaren Materials auf ein Gipsmodell und Polymerisation des Materials zu schaffen, das es erlaubt, diese Geräte und Apparaturen mit hoher Detailgenauigkeit und unter Verwendung eines gebrauchsfertigen Einkomponenten-Materials mit praktisch unbegrenzter Verarbeitungszeit anzufertigen.

Die Lösung der Aufgabe ist ein Verfahren zur Herstellung von kieferorthopädischen Geräten und Apparaturen durch Aufbringen eines 40—80 Gewichts-% des aus 2,2,4 - Trimethylhexamethylendiisocyanat und 2 - Hydroxyäthylmethacrylat hergestellten Diurethandimethacrylats und einen Photopolymerisationskatalysator enthaltenden Materials in dünner Schicht auf ein Gipsmodell, teilweise Polymerisation des Materials bis zu gelartiger Konsistenz durch kurzzeitige Bestrahlung mit Licht einer Wellenlänge von 400—450 nm, Wiederholen von schichtweisem Aufbringen und kurzzeitiger Bestrahlung so lange, bis die für das Gerät beziehungsweise die Apparatur gewünschte Schichtdicke und Form erreicht ist, und ausschließende vollständige Polymerisation des teilweise polymerisierten Materials durch Bestrahlung mit Licht einer Wellenlänge von 350 nm.

Das erfindungsgemäße Verfahren hat sich besonders bewährt, wenn ein Material, das neben mindestens 40 Gewichts-%, vorzugsweise 40—80 Gewichts-%, des genannten Diurethandimethacrylats ein Prepolymer auf Basis eines acrylierten Polyurethans oder ein Prepolymer auf Basis eines acrylierten Epoxidharzes und/oder Bis - [4 - (2 - hydroxy - 3 - methacryloyloxypropoxyphenyl] - dimethyl - methan—auch als Bis-GMA bezeichnet—und einen oder mehrere niedrigviskose, auch als reaktive Verdünnungsmittel oder verdünnende Monomere bezeichnete Acrylsäureester und/oder Methacrylsäureester enthält, eingesetzt wird.

Als reaktive Verdünnungsmittel können alle bekannten Acryl- und Methacrylsäureester dieser Art verwendet werden.

Besonders bewährt haben sich 2 - Hydroxyäthylacrylat, Tripropylenglykoldiacrylat, Pentaerythrittetraacrylat, Butoxyäthylacrylat, 2 - Hydroxyäthylmethacrylat, Butandioldimethacrylat, Triäthylenglykoldimethacrylat und Dodecandioldimethacrylat.

Durch den Zusatz der Prepolymeren und/oder des Bis-GMA und der niedrigviskosen Acryl- und Methacrylsäureester wird ein Material erhalten, das aufgrund seines Fließverhaltens die Modelloberfläche mit ihren Vertiefungen und Erhöhungen getreu nachbildet und Klammern, Drähte, Dehnungsschrauben und dergleichen dicht umhüllt.

Durch die erste kurzzeitige—etwa 5 bis 10 Sekunden dauernde—Bestrahlung mit Licht einer Wellenlänge von 400—450 nm bildet sich aus dem schichtförmig aufgebrachten, eine honigartige Konsistenz besitzenden Material eine tragfähige Schicht von gelartiger Konsistenz, auf die—ohne sie zu verändern—weiteres Material aufgebracht werden kann.

Infolge der getreuen Nachbildung der Modelloberfläche, der ein genaues Arbeiten ermöglichenden Transparenz des Materials und der auf der Art des schichtweisen Aufbaus und der teilweisen Polymerisation beruhenden geringen Volumenänderung (Schrumpfung durch Polymerisation) ist die Paßgenauigkeit und Dimensionstreue der nach dem erfindungsgemäßen Verfahren hergestellten kieferorthopädischen Geräte und Apparaturen sehr gut.

Abgesehen von den Klammern, Drähten, Dehnungsschrauben und dergleichen sind die nach dem erfindungsgemäßen Verfahren hergestellten kieferorthopädischen Geräte und Apparaturen transparent und farblos oder—falls ein mit einem geeigneten Farbstoff gefärbtes Material benutzt wurde—farbig.

Der in dem Material vorhandene Photopolymerisationskatalysator kann irgendein bekannter Photoinitiator für die Photopolymerisation von Acryl- und Methacrylsäureestern sein.

Bewährt haben sich Photoinitiatoren aus Ketonen und Reduktionsmitteln, Bevorzugt werden Gemische Aus Campherchinon und Aminen, zum Beispiel Methyldiäthanolamin oder Triäthanolamin, besonders solche, die zusätzlich noch als Katalysatoren für die Photopolymerisation bekannte Benzoylverbindungen, zum Beispiel Benzilacetale und Benzoylalkanole, enthalten.

Zur näheren Erläuterung wird in den folgenden Beispielen die Herstellung eines kieferorthopädischen Gerätes nach dem erfindungsgemäßen Verfahren und das dazu benutzte Material, das vorzugsweise in lichtundurchlässige Schraubenpressen oder Spritzen verpackt wird, beschrieben.

Beispiel 1
Herstellung eines kieferorthopädischen Gerätes

In dünner Schicht wird das Material auf das vorbereitete, isolierte Gipsmodell aufgebracht, wobei die verwendeten Klammern und Drähte sorgfältig umspült werden. Das Material wird dann durch kurzzeitige Bestrahlung (5—10 sec.) mit Licht einer Wellenlänge von 400—450 nm teilweise polymerisert, wobei es eine gelartige Konsistenz bekommt. Dann wird nach und nach weiteres Material in Schichten aufgebracht und—wie beschrieben—durch kurzzeitige Bestrahlung teilweise polymerisiert. Ist Form und Gestalt des gewünschten Gerätes .erreicht, so wird das auf dem Gipsmodell befindliche teilweise polymerisierte Material 2 Minuten long und—nach Abnehmen von Dem Gipsmodell—noch 1 Minute lang von der Gegenseite her mit Licht einer Wellenlänge von 350 nm bestrahlt. Das so hergestellte kieferorthopädische Gerät kann, falls es erforderlich sein sollte, in üblicher Weise nachbearbeitet werden.

Die Bestrahlung erfolgt mit handelsüblichen Halogenleuchten ausreichender Energie, zum Beispiel mit dem Wolfram - Halogen - Lichtgerät Translux der Firma Kulzer, und UV-Strahlenquellen, insbesondere mit einer Quecksilber - Hochdrucklampe.

Beispiel 2
Polymerisierbares Material

| A) | Gewichts-% | Diurethandimethacrylat, hergestellt aus 1 mol 2,2,4 - Trimethylhexamethylen-diisocyanat und 2 mol 2 - Hydroxyäthylmethacrylat |
| | 37,5 Gewichts-% | Prepolymer auf Basis eines acrylierten Epoxidharzes der Firma Degussa, Frankfurt, mit der Bezeichnung VPS 1928 |
| | 20 Gewichts-% | Triäthylenglykoldimethacrylat |
| | 0,2 Gewichts-% | Camperchinon |
| | 0,5 Gewichts-% | 1,2 - Diphenyl - 2,2 - dimethoxyäthanon |
| | 1 Gewichts-% | 1 - (4 - Dodecylphenyl) - 2 - hydroxy - 2 - methylpropanon |
| | 0,8 Gewichts-% | Triäthanolamin |
| B) | 60 Gewichts-% | Diurethandimethacrylat, hergestellt aus 1 mol 2,2,4 - Trimethylhexamethylen-diisocyanat und 2 mol 2 - Hydroxyäthylmethacrylat |
| | 17,5 Gewichts-% | Prepolymer auf Basis eines acrylierten Epoxidharzes der Firma Degussa, Frankfurt, mit der Bezeichnung VPS 1940 |
| | 10 Gewichts-% | Bis-GMA |
| | 10 Gewichts-% | Dodecandioldimethacrylat |
| | 0,2 Gewichts-% | Camperchinon |
| | 0,5 Gewichts-% | 1,2 - Diphenyl - 2,2 - dimethoxyäthanon |
| | 1 Gewichts-% | 1 - (4 - Dodecylphenyl) - 2 - hydroxy - 2 - methylpropanon |
| | 0,8 Gewichts-% | Triäthanolamin |

EP 0 151 686 B1

| C) | 75 Gewichts-% | Diurethandimethacrylat, hergestellt aus 1 mol 2,2,4 - Trimethylhexamethylen-diisocyanat und 2 mol 2 - Hydroxyäthylmethacrylat |
| | 2,5 Gewichts-% | Prepolymer auf Basis eines acrylierten Epoxidharzes der Firma Degussa, Frankfurt, mit der Bezeichnung VPS 1940 |
| | 5 Gewichts-% | Bis-GMA |
| | 5 Gewichts-% | Pentaerythrittetraacrylat |
| | 10 Gewichts-% | Triäthylenglykoldimethacrylat |
| | 0,2 Gewichts-% | Campherchinon |
| | 0,5 Gewichts-% | 1,2 - Diphenyl - 2,2 - dimethoxyäthanon |
| | 1 Gewichts-% | 1 - (4 - Dodecylphenyl) - 2 - hydroxy - 2 - methylpropanon |
| | 0,8 Gewichts-% | Triäthanolamin |

| D) | 70 Gewichts-% | Diurethandimethacrylat, hergestellt aus 1 mol 2,2,4 - Trimethylhexamethylen-diisocyanat und 2 mol 2 - Hydroxyäthylmethacrylat |
| | 12,5 Gewichts-% | Prepolymer auf Basis eines acrylierten Polyurethans der Firma Degussa, Frankfurt, mit der Bezeichnung VPS 1748 |
| | 5 Gewichts-% | Bis-GMA |
| | 10 Gewichts-% | Triäthylenglykoldimethacrylat |
| | 0,5 Gewichts-% | Campherchinon |
| | 1 Gewichts-% | 1 - (4 - Dodecylphenyl) - 2 - hydroxy - 2 - methylpropanon |
| | 1 Gewichts-% | Tripropanolamin |

| E) | 80 Gewichts-% | Diurethandimethacrylat, hergestellt aus 1 mol 2,2,4 - Trimethylhexamethylen-diisocyanat und 2 mol 2 - Hydroxyäthylmethacrylat |
| | 7,48 Gewichts-% | Bis-GMA |
| | 10 Gewichts-% | Dodecandioldimethacrylat |
| | 0,02 Gewichts-% | Macrolexrot der Firma Bayer, Leverkusen |
| | 0,2 Gewichts-% | Campherchinon |
| | 0,5 Gewichts-% | 1,2 - Diphenyl - 2,2 - dimethoxyäthanon |
| | 1,0 Gewichts-% | 1 - (4 - Dodecylphenyl) - 2 - hydroxy - 2 - methylpropanon |
| | 0,8 Gewichts-% | Triäthanolamin |

**Patentansprüche**

1. Verfahren zur Herstellung von kieferorthopädischen Geräten und Apparaturen durch Aufbringen eines 40—80 Gewichts-% des aus 2,2,4 - Trimethylhexamethylendiisocyanat und 2 - Hydroxyäthylmethacrylat hergestellten Diurethandimethacrylats und eine Photopolymerisationskatalysator enthaltenden Materials in dünner Schicht auf ein Gipsmodell, teilweise Polymerisation des Materials bis zu gelartiger Konsistenz durch kurzzeitige Bestrahlung mit Licht einer Wellenlänge von 400—450 nm, Wiederholen von schichtweisem Aufbringen und kurzzeitiger Bestrahlung so lange, bis die für das Gerät beziehungsweise die Apparatur gewünschte Schichtdicke und Form erreicht ist, und anschließende vollständige Polymerisation des teilweise polymerisierten Materials durch Bestrahlung mit Licht einer Wellenlänge von 350 nm.

2. Verfahren nach Anspruch 1, dadurch gekennzeichnet, daß das Material zusätzlich ein Prepolymer auf Basis eines acrylierten Polyurethans oder ein Prepolymer auf Basis eines acrylierten Epoxidharzes und/oder Bis - [4 - (2 - hydroxy - 3 - methacryloyloxypropoxyphenyl] - dimethylmethan enthält.

3. Verfahren nach Anspruch 1 oder 2, dadurch gekennzeichnet, daß das Material zusätzlich einen oder mehrere niedrigviskose Acrylsäureester und/oder Methacrylsäureester enthält.

4. Verfahren nach einem der Ansprüche 1 bis 3, dadurch gekennzeichnet, daß das Material als Photopolymerisationskatalysator ein Gemisch aus einem oder mehreren Ketonen und einem Amin enthält.

5. Verfharen nach Anspruch 4, dadurch gekennzeichnet, daß der Photopolymerisationskatalysator ein Gemisch aus Campherchinon und einem Amin ist.

6. Verfahren nach Anspruch 5, dadurch gekennzeichnet, daß der Photopolymerisationskatalysator zusätzlich mindestens eine Benzoylverbindung enthält.

**Revendications**

1. Procédé pour la fabrication de dispositifs et appareils orthopédiques maxillaires par application en couche mince sur un modèle de plâtre d'une matière contenant 40—80% en poids du diuréthannediméth-acrylate fabriqué à partir de 2,2,4 - trimethylhexaméthylènediisocyanate et de méthacrylate de 2 - hydroxyéthyle et un catalyseur de photopolymérisation, polymérisation partielle de la matière jusqu'à une consistance gélatineuse par irradiation de courte durée par une lumière d'une longueur d'onde de 400—450 nm, répétition de l'application en couche et de l'irradiation de courte durée jusqu'à ce que l'épaisseur de couche et la forme souhaitées pour le dispositif ou l'appareil soient atteintes et ensuite

4

polymérisation complète de la matière partiellement polymérisée, par irradiation par de la lumière d'une longueur d'onde de 350 nm.

2. Procédé selon la revendication 1, caractérisé en ce que la matière contient en outre un prépolymère à base d'un polyuréthanne acrylé ou un prépolymère à base d'une résine époxydique acrylée et/ou du bis - [4 - (2 - hydroxy - 3 - méthacryloyloxypropoxy)phényl] - diméthylméthane.

3. Procédé selon la revendication 1 ou 2, caractérisé en ce que la matière contient en outre un ou plusieurs esters acryliques et/ou méthacryliques de faible viscosité.

4. Procédé selon l'une des revendications 1 à 3, caractérisé en ce que la matière contient comme catalyseur de photopolymérisation un mélange d'une ou plusieurs cétones et d'une amine.

5. Procédé selon la revendication 4, caractérisé en ce que le catalyseur de photopolymérisation est un mélange de camphoquinone et d'une amine.

6. Procédé selon la revendication 5, caractérisé en ce que le catalyseur de photopolymérisation contient en outre au moins un composé benzoylé.

**Claims**

1. Process for the production of jaw orthopaedic appliances and apparatuses by application in a thin layer to a plaster model of a material containing (a) 40—80 weight % of the diurethane dimethacrylate prepared from 2,2,4 - trimethylhexamethylene diisocyanate and 2 - hydroxyethyl methacrylate and (b) a photopolymerization catalyst, partial polymerization of the material to a gelatinous consistency by brief irradiation with light of a wavelength of 400—450 nm, repetition of application by layers and brief irradiation until the layer thickness and form desired for the appliance or the apparatus have been achieved, and subsequent complete polymerization of the partially polymerized material by irradiation with light of a wavelength of 350 nm.

2. Process according to Claim 1 characterized in that the material additionally contains a prepolymer based on an acrylated polyurethane or a prepolymer based on an acrylated epoxy resin and/or bis - [4 - hydroxy - 3 - methacryloyloxypropoxyphenyl] - dimethylmethane.

3. Process according to Claim 1 or 2, characterized in that the material additionally contains one or more low-viscosity acrylic acid esters and/or methacrylic acid esters.

4. Process according to one of Claims 1 to 3, characterized in that the material contains as photopolymerization catalyst a mixture of one or more ketones and an amine.

5. Process according to Claim 4, characterized in that the photopolymerization catalyst is a mixture of camphor quinone and an amine.

6. Process according to Claim 5, characterized in that the photopolymerization catalyst contains in addition at least one benzoyl compound.